# EUROPEAN PATENT SPECIFICATION

(11) **EP 2 971 107 B1**
(45) Date of publication and mention of the grant of the patent: **24.04.2019**
(21) Application number: 14724567.4
(22) Date of filing: 14.03.2014
(51) Int. Cl.: C12Q 1/6886

(54) **DIAGNOSTIC AND PROGNOSTIC MARKER FOR PROSTATE CANCER**
DIAGNOSTISCHER UND PROGNOSTISCHER MARKER FÜR PROSTATAKREBS
MARQUEUR DE DIAGNOSTIC ET DE PRONOSTIC POUR LE CANCER DE LA PROSTATE

(30) Priority: 15.03.2013 US 201361793413 P
(43) Date of publication of application: 20.01.2016
(73) Proprietor: Onconostic Technologies, Inc., San Diego, California 92130 (US)
(72) Inventor: RAY, Partha S., Champaign Illinois 62822 (US)
(74) Representative: Schiweck Weinzierl Koch Patentanwälte Partnerschaft mbB
(86) International application number: PCT/US2014/027912
(87) International publication number: WO 2014/143794

(56) References cited:
- LEONIE VAN DER HEUL-NIEUWENHUIJSEN ET AL: "Gene expression of forkhead transcription factors in the normal and diseased human prostate", BJU INTERNATIONAL, vol. 103, no. 11, 1 June 2009 (2009-06-01) , pages 1574-1580, XP055140131, ISSN: 1464-4096, DOI: 10.1111/j.1464-410X.2009.08351.x
- THORAT M A ET AL: "Forkhead box A1 expression in breast cancer is associated with luminal subtype and good prognosis.", JOURNAL OF CLINICAL PATHOLOGY MAR 2008, vol. 61, no. 3, March 2008 (2008-03), pages 327-332, XP008172091, ISSN: 1472-4146

## Description

### TECHNICAL FIELD

The present disclosure is generally related to methods for determining a diagnosis and/or prognosis for prostate cancer.

### TECHNICAL BACKGROUND

Van der Heul-Nieuwenhuijsen et al. (2009), BJU International, Vol. 103(11), pages 1574-1580, disclose an analysis of the expression pattern of 12 different FOX genes in normal and diseased prostate cell lines. Van der Heul-Nieuwenhuijsen et al. are however completely silent as to monitoring prognosis of prostate cancer and correlating the prognosis with the ratio of FOXC1:FOXA1, let alone any disclosure of a particular cut off value for the ratio. Thorat et al. (2008), Journal of Clinical Pathology, Vol 61(3), pages 327-332 disclose the prognosis of breast cancer patients but fail to disclose that the ratio of FOXC1:FOXA1 correlates with the prognosis of prostate cancer.

### SUMMARY OF THE INVENTION

The present invention is based on the discovery that the FOXC1:FOXA1 ratio is predictive of a long-term outcome in patients diagnosed as having a prostate cancer. Accordingly, the FOXC1:FOXA1 ratio may be used to diagnose a prostate cancer, distinguish a prostate cancer from benign prostatic hyperplasia (BPH), determine a prognosis for a prostate cancer, and/or develop and implement a treatment plan for a patient diagnosed as having a prostate cancer.

In one aspect, the invention provides a method for determining the prognosis of a prostate cancer in a subject diagnosed as having prostate cancer, comprising:
(i) determining in a tissue sample (e.g., prostate tissue) from said subject, wherein said tissue sample comprises prostate cancer cells, the level of FOXA1 protein or nucleic acid, wherein the FOXA1 nucleic acid is optionally FOXA1 mRNA;
(ii) determining the level of FOXC1 protein or nucleic acid in said tissue sample, wherein the FOXC1 nucleic acid is optionally FOXC1 mRNA,
(iii) calculating the ratio of said FOXC1 protein or nucleic acid to said FOXA1 protein or nucleic acid;
(iv) comparing said ratio to a comparable FOXC1:FOXA1 ratio in a reference population of samples comprising prostate cancer cells in which a prognostic outcome is associated with each of the reference population samples; and
(v) identifying said subject as having a poor prognosis relative to the prognostic outcome when the FOXC1 :FOXA1 ratio of the subject is equal to or greater than a FOXC1:FOXA1 cutoff ratio, and identifying said subject as having a good prognosis relative to the prognostic outcome when the FOXC1 :FOXA1 ratio of the subject is less than the FOXC1:FOXA1 cutoff ratio, wherein the FOXC1:FOXA1 cutoff ratio corresponds to at least the 50^{th} percentile of FOXC1:FOXA1 ratios of the reference population.

In some embodiments of this aspect, the FOXC1:FOXA1 cutoff ratio used to determine the prognosis corresponds to the value associated with the 50^{th}, 60^{th}, 70^{th}, 75^{th}, 80^{th}, 90^{th}, 95^{th}, or even 99^{th} percentile of FOXC1:FOXA1 ratios of the reference population of samples comprising prostate cancer cells.

In some embodiments of this aspect, the method further comprises: (vi) developing, implementing, and/or modifying a treatment plan based on the prognostic identification of step (v). Optionally, the treatment plan includes discontinuing, maintaining, initiating, or modifying at least one anti-cancer therapy for a subject having a good prognosis or for a subject having a poor prognosis. Modifications to an anti-cancer therapy may include altering (i.e., increasing or decreasing) the frequency, duration, or dose of that anti-cancer therapy. The initiation of an anti-cancer therapy may include adding a previously unused chemotherapeutic agent to the treatment regimen and/or performing a surgical resection of the prostate, surrounding tissues, and/or distant tissues in which metastasis are expected or confirmed.

In another aspect, the invention provides a method for diagnosing a prostate cancer in a subject, comprising:
(i) determining in a tissue sample (e.g., prostate tissue) from said subject, wherein said tissue sample is suspected of comprising prostate cancer cells, the level of FOXA1 protein or nucleic acid, wherein the FOXA1 nucleic acid is optionally FOXA1 mRNA;
(ii) determining the level of FOXC1 protein or nucleic acid in said tissue sample, wherein the FOXC1 nucleic acid is optionally FOXC1 mRNA,
(iii) calculating the ratio of said FOXC1 protein or nucleic acid to said FOXA1 protein or nucleic acid;
(iv) comparing said ratio to a comparable FOXC1:FOXA1 ratio in a reference population of samples known to comprise prostate cancer cells; and
(v) identifying said subject as having a prostate cancer when the FOXC1:FOXA1 ratio of the subject is equal to or greater than a FOXC1:FOXA1 cutoff ratio, and identifying said subject as not having a prostate cancer when the FOXC1:FOXA1 ratio of the subject is less than the FOXC1:FOXA1 cutoff ratio, wherein the FOXC1:FOXA1 cutoff ratio corresponds to at least the 20^{th} percentile of FOXC1:FOXA1 ratios of the reference population.

In some embodiments of this aspect, the FOXC1:FOXA1 cutoff ratio used to determine a diagnosis corresponds to the value associated with the 20^{th}, 25^{th}, 30^{th}, 40^{th}, 50^{th}, 60^{th}, 70^{th}, 75^{th}, 80^{th}, 90^{th}, 95^{th}, or even 99^{th} percentile of FOXC1:FOXA1 ratios of the reference population of samples comprising prostate cancer cells, lack prostate cancer cells, or a mixed population in which some samples comprise prostate cancer cells and other samples lack prostate cancer cells.

In some embodiments of this aspect, the method further comprises: (vi) developing and implementing a treatment plan based on the diagnostic identification of step (v). Optionally, the treatment plan includes initiating at least one anti-cancer therapy including, but not limited to, initiating a chemotherapy, initiating a radiation therapy, and surgical resection of the prostate, surrounding tissues, and/or distant tissues in which metastasis are expected or confirmed.

In another aspect, the disclosure provides a method for determining and implementing a treatment plan in a subject diagnosed as having a prostate cancer, said method comprising:
(i) obtaining a tissue sample (e.g., prostate tissue) from said subject, wherein said tissue sample comprises prostate cancer cells;
(ii) determining the level of FOXA1 protein or nucleic acid in said tissue sample,
(iii) determining the level of FOXC1 protein or nucleic acid in said tissue sample,
(iv) calculating the ratio of said FOXC1 protein or nucleic acid to said FOXA1 protein or nucleic acid;
(v) comparing said ratio to a comparable FOXC1:FOXA1 ratio in a reference population of samples known to comprise prostate cancer cells; and
(vi) developing and implementing a treatment plan based on the comparison of step (v).

The treatment plan may include any one or more of the following: discontinuing, maintaining, initiating, or modifying at least one anti-cancer therapy for the subject. Modifications to an anti-cancer therapy may include altering (i.e., increasing or decreasing) the frequency, duration, or dose of that anti-cancer therapy. The initiation of an anti-cancer therapy may include adding a previously unused chemotherapeutic agent to the treatment regimen and/or performing a surgical resection of the prostate, surrounding tissues, and/or distant tissues in which metastasis are expected or confirmed.

In another aspect, the disclosure provides a method for distinguishing between a prostate cancer and benign prostatic hyperplasia (BPH) in a subject, comprising:
(i) obtaining a tissue sample (e.g., prostate tissue) from said subject;
(ii) determining the level of FOXA1 protein or nucleic acid in said tissue sample,
(iii) determining the level of FOXC1 protein or nucleic acid in said tissue sample,
(iv) calculating the ratio of said FOXC1 protein or nucleic acid to said FOXA1 protein or nucleic acid;
(v) comparing said ratio to a comparable FOXC1:FOXA1 ratio in a reference population of samples comprising the FOXC1 :FOXA1 ratio from samples known to be obtained from subjects having a prostate cancer and/or samples know to be obtained from subjects having BPH; and
(vi) identifying said subject as having a prostate cancer when the FOXC1:FOXA1 ratio of the subject is equal to or greater than a FOXC1:FOXA1 cutoff ratio that is indicative of prostate cancer, or identifying said subject as not having BPH when the FOXC1:FOXA1 ratio of the subject is less than the FOXC1:FOXA1 cutoff ratio that is indicative of prostate cancer.

In some embodiments of this aspect, the FOXC1:FOXA1 cutoff ratio used to determine a diagnosis corresponds to the value associated with the 1^{st}, 5^{th}, 10^{th}, 20^{th}, 25^{th}, 30^{th}, 40^{th}, 50^{th}, 60^{th}, 70^{th}, 75^{th}, 80^{th}, 90^{th}, 95^{th}, or even 99^{th} percentile of FOXC1:FOXA1 ratios of the reference population of samples comprising prostate cancer cells, lack prostate cancer cells, a mixed population in which some samples comprise prostate cancer cells and other samples lack prostate cancer cells, and/or were obtained from subjects known to have BPH but not prostate cancer.

In some embodiments of any of the foregoing aspects, either of both of the FOXA1 and FOXC1 nucleic acid is assessed in the sample and the nucleic acid is RNA (e.g., mRNA). Optionally, the RNA is assessed directly or in the form of cDNA which optionally may be amplified (e.g., by reverse transcriptase PCR).

In some embodiments of any of the foregoing aspects, the subject also may be assessed for additional prostate cancer markers or indicators including, for example, (i) the presence of a TMRPSS2:ERG translocation in prostate cancer cells, (ii) the presence, absence, or amount of an estrogen receptor (ER) or ER subtype (e.g., ERα and/or ERβ) in prostate cancer cells, (iii) the presence, absence, or amount of an androgen receptor or androgen receptor subtype in the prostate cancer cells, and (iv) the presence, absence, or amount (absolute or relative) of prostate-specific antigen (PSA). Such an assessment optionally may be used in determining the appropriate action (e.g., determining a diagnosis, prognosis, or treatment plan).

In some embodiments of any of the foregoing aspects, any suitable reference population may be used for the comparison of the subject's FOXC1:FOXA1 ratio. Useful reference populations include populations comprising or limited to (i) samples of the specific prostate cancer subtype as identified in the subject (e.g., adenocarcinoma), (ii) samples having the same TMPRSS2:ERG translocation status (e.g., presence or absence) as the subject, and (iii) samples having ER status (e.g., positive or negative for ER generally, or for any specific ER subtype).

When comparing the FOXC1 :FOXA1 ratio to the FOXC1 :FOXA1 cutoff ratio determined from the reference population, a subject's FOXC1 :FOXA1 ratio that is greater than or less than the FOXC1:FOXA1 cutoff ratio and indicates the particular action (diagnosis, prognosis, treatment plan) depending upon which specific action is under consideration and the makeup of the reference population against which the subject's FOXC1:FOXA1 ratio is being compared. For example, when determining a prognosis for a subject and comparing the subject's FOXC1:FOXA1 ratio against a reference population of prostate cancer samples, FOXC1:FOXA1 ratios less than the chosen cutoff ratio is indicative of a better prognosis relative to FOXC1:FOXA1 ratios that are greater than the chosen cutoff ratio.

In another aspect, the disclosure provides a method for determining the prognosis of a prostate cancer in a subject diagnosed as having prostate cancer, comprising:
(i) obtaining a tissue sample (e.g., prostate tissue) from said subject, wherein said tissue sample comprises, or is suspected of comprising, prostate cancer cells;
(ii) determining the level of FOXC1 protein or nucleic acid in said tissue sample,
(iii) comparing said level of FOXC1 to the level of FOXC1 cutoff level in a reference population of samples comprising prostate cancer cells in which a prognostic outcome is associated with each of the reference population samples; and
(vi) identifying said subject as having a poor prognosis relative to the prognostic outcome when the level of FOXC1 of the subject is equal to or greater than a FOXC1 cutoff level, and identifying said subject as having a good prognosis relative to the prognostic outcome when the level of FOXC1 of the subject is less than the FOXC1 cutoff level, wherein the FOXC1 cutoff level corresponds to at least the 50^{th} percentile of FOXC1 levels of the reference population.

In some embodiments of this aspect, the FOXC1 cutoff level used to determine the prognosis corresponds to the value associated with the 25^{th}, 30^{th}, 40^{th}, 50^{th}, 60^{th}, 70^{th}, 75^{th}, 80^{th}, 90^{th}, 95^{th}, or even 99^{th} percentile of FOXC1 levels of the reference population of samples comprising prostate cancer cells.

In some embodiments of this aspect, the method further comprises: (vii) developing, implementing, and/or modifying a treatment plan based on the prognostic identification of step (iv). Optionally, the treatment plan includes discontinuing, maintaining, initiating, or modifying at least one anti-cancer therapy for a subject having a good prognosis or for a subject having a poor prognosis. Modifications to an anti-cancer therapy may include altering (i.e., increasing or decreasing) the frequency, duration, or dose of that anti-cancer therapy. The initiation of an anti-cancer therapy may include adding a previously unused chemotherapeutic agent to the treatment regimen and/or performing a surgical resection of the prostate, surrounding tissues, and/or distant tissues in which metastasis are expected or confirmed.

In another aspect, the disclosure provides a method for diagnosing a prostate cancer in a subject, comprising:
(i) obtaining a tissue sample (e.g., prostate tissue) from said subject, wherein said tissue sample is suspected of comprising prostate cancer cells;
(ii) determining the level of FOXC1 protein or nucleic acid in said tissue sample,
(iii) comparing said level of FOXC1 to a comparable FOXC1 cutoff level in a reference population of samples known to comprise prostate cancer cells; and
(vi) identifying said subject as having a prostate cancer when the FOXC1 level of the subject is equal to or greater than a FOXC1 cutoff level , and identifying said subject as not having a prostate cancer when the FOXC1 level of the subject is less than the FOXC1 cutoff level, wherein the FOXC1 cutoff level corresponds to at least the 20^{th} percentile of FOXC1 levels of the reference population.

In some embodiments of this aspect, the FOXC1 cutoff level is used to determine a diagnosis corresponds to the value associated with the 1^{st}, 5^{th}, 10^{th}, 20^{th}, 25^{th}, 30^{th}, 40^{th}, 50^{th}, 60^{th}, 70^{th}, 75^{th}, 80^{th}, 90^{th}, 95^{th}, or even 99^{th} percentile of FOXC1 levels of the reference population of samples comprising prostate cancer cells, lack prostate cancer cells, or a mixed population in which some samples comprise prostate cancer cells and other samples lack prostate cancer cells.

In some embodiments of this aspect, the method further comprises: (vii) developing and implementing a treatment plan based on the diagnostic identification of step (iv). Optionally, the treatment plan includes initiating at least one anti-cancer therapy including, but not limited to, initiating a chemotherapy, initiating a radiation therapy, and surgical resection of the prostate, surrounding tissues, and/or distant tissues in which metastasis are expected or confirmed.

In another aspect, the disclosure provides a method for determining and implementing a treatment plan in a subject diagnosed as having a prostate cancer, said method comprising:
(i) obtaining a tissue sample (e.g., prostate tissue) from said subject, wherein said tissue sample comprises prostate cancer cells;
(ii) determining the level of FOXC1 protein or nucleic acid in said tissue sample,
(iii) comparing said level of FOXC1 in the subject to a comparable FOXC1 cutoff level in a reference population of samples known to comprise prostate cancer cells; and
(iv) developing and implementing a treatment plan based on the comparison of step (iii).

The treatment plan may include any one or more of the following: discontinuing, maintaining, initiating, or modifying at least one anti-cancer therapy for the subject. Modifications to an anti-cancer therapy may include altering (i.e., increasing or decreasing) the frequency, duration, or dose of that anti-cancer therapy. The initiation of an anti-cancer therapy may include adding a previously unused chemotherapeutic agent to the treatment regimen and/or performing a surgical resection of the prostate, surrounding tissues, and/or distant tissues in which metastasis are expected or confirmed.

In another aspect, the disclosure provides a method for distinguishing between a prostate cancer and benign prostatic hyperplasia (BPH) in a subject, comprising:
(i) obtaining a tissue sample (e.g., prostate tissue) from said subject;
(ii) determining the level of FOXC1 protein or nucleic acid in said tissue sample,
(iii) comparing said level of FOXC1 to a comparable FOXC1 cutoff level in a reference population of samples comprising the FOXC1 level from samples known to be obtained from subjects having a prostate cancer and/or samples know to be obtained from subjects having BPH; and
(iv) identifying said subject as having a prostate cancer when the FOXC1 level of the subject is equal to or greater than a FOXC1 cutoff level that is indicative of prostate cancer, or identifying said subject as having BPH when the FOXC1 level of the subject is less than the FOXC1 cutoff level that is indicative of prostate cancer.

In some embodiments of this aspect, the FOXC1 cutoff level used to determine a diagnosis corresponds to the value associated with the 1^{st}, 5^{th}, 10^{th}, 20^{th}, 25^{th}, 30^{th}, 40^{th}, 50^{th}, 60^{th}, 70^{th}, 75^{th}, 80^{th}, 90^{th}, 95^{th}, or even 99^{th} percentile of FOXC1 levels of the reference population of samples comprising prostate cancer cells, lack prostate cancer cells, a mixed population in which some samples comprise prostate cancer cells and other samples lack prostate cancer cells, and/or were obtained from subjects known to have BPH but not prostate cancer.

In some embodiments of any of the foregoing aspects, either of both of the FOXA1 and FOXC1 nucleic acid is assessed in the sample and the nucleic acid is RNA (e.g., mRNA). Optionally, the RNA is assessed directly or in the form of cDNA which optionally may be amplified (e.g., by reverse transcriptase PCR).

In some embodiments of any of the foregoing aspects, the prostate cancer is an adenocarcinoma, a small cell carcinoma, or a prostatic sarcoma.

When multiple markers are assessed in a single subject, they may be assessed in the same or different samples or sample types. For example, FOXA1, FOXC1, and ER may be assessed in prostate tissue samples while PSA may be assessed in a blood sample.

By "prostate cancer" is meant any cancer of the prostate of any cell type including, but not limited to, adenocarcinoma, small cell carcinoma, and prostatic sarcoma.

By "FOXA1" is meant any protein or nucleic acid, as the context demands, that is commonly known as FOXA1 or the forkhead box protein A1, as described herein. It is recognized that there is a certain amount of variability in known human FOXA1 sequences. Thus, without limitation, exemplary FOXA1 protein sequences include those found at GenBank accession Nos.: EAW65844.1 GI: 119586248; and AAH33890.1 GI: 119586248. Exemplary FOXA1 nucleic acid sequences may be found at FOXA1 RefSeqGene on chromosome 14 (NG_033028.1 GI: 429836885). Exemplary mRNA sequences include Accession Nos: NM_004496.3 GI: 385298683; BC033890.1 GI: 21707516; and AK313785.1 GI: 164695568.

By "FOXC1" is meant any protein or nucleic acid, as the context demands, that is commonly known as FOXC1 or the forkhead box protein C1, as described herein. It is recognized that there is a certain amount of variability in known human FOXC1 sequences. Thus, without limitation, exemplary FOXC1 protein sequences include those found at GenBank accession Nos.: AAI34422.1 GI: 126632009; and NP_001444.2 GI: 119395716. Exemplary FOXA1 nucleic acid sequences may be found at FOXC1 RefSeqGene on chromosome 6 (NG_009368.1 GI: 221139902); and AY228704.1 GI: 30143279; AY228705.1 GI: 30143281; and KF855955.1 GI: 584292399. Exemplary mRNA sequences include Accession Nos: NM_001453.2 GI: 119395715.

A "prognostic outcome" refers to any endpoint that is directly or indirectly related to prostate cancer and its associated conditions that is useful for measuring, for example, the effectiveness of therapy, disease progression, or other clinical outcome. Useful prognostic outcomes include survival duration (i.e., mortality) which may be measured either as a binary event at any given time point (e.g., 5-year survival) or as a function of time (e.g., expressed as a survival curve over time), event-free survival, and relapse-free survival (e.g., relapse rate).

A "good prognosis," with reference to a particular prognostic outcome and specific FOXC1:FOXA1 ratio, refers to a prediction of that outcome in that subject will be more favorable than the expected outcome for individuals having a different (less favorable) FOXC1:FOXA1 ratio or than the average outcome for all patients diagnosed as having that type or subtype of prostate cancer, regardless of FOXC1:FOXA1 ratio. Likewise, a "poor prognosis" refers to a prediction of that outcome in that subject will be less favorable than the expected outcome for individuals having a different (more favorable) FOXC1:FOXA1 ratio or than the average outcome for all patients diagnosed as having that type or subtype of prostate cancer, regardless of FOXC1:FOXA1 ratio. The specific outcomes are, of course, dependent upon the specific prognostic outcome under consideration.

"Developing a treatment plan" refers to the process engaged by a physician in determining the appropriate course of therapy (or non-therapy) for a particular subject. The development of a treatment plan may be based solely on the measured FOXC1:FOXA1 ratio in that subject or may include other demographic information (e.g., age, ethnicity, etc..) and/or medical history (e.g., history of prior therapy). Developing a treatment plan may include initiating a new therapy, discontinuing a therapy, or modifying an existing therapeutic regimen. Such modifications may include altering the frequency, duration, dose, and/or route of administration of an existing therapy.

"Determining the level," with reference to an assessment of any relevant biological marker including FOXA1 and FOXC1, means obtaining an informative assessment of that marker in the subject or any relevant biological sample obtained from the subject. The assessment may be limited to determining the presence or absence of a marker, qualitative assessments (e.g., pathological assessments such as determining the cellular or sub-cellular localization of the marker), or may include semi-quantitative assessments and/or quantitative assessments.

"Anti-cancer therapy," with reference to prostate cancer, means any useful or experimental treatment regimen or procedure designed to reduce or eliminate the cancer and/or provide a palliative effect. Anti-cancer therapy includes chemotherapy, radiation therapy, and surgery (e.g., resection of the tumor). Surgical resection (complete or partial) may include resection of prostate tissue, surrounding tissue, and/or distant tissues in which metastasis are suspected or confirmed (e.g., lymph nodes).

"Tissue sample" refers to any liquid, solid, or mixed biological sample obtained from a subject having, or suspected of having, prostate cancer which is useful for the assessment of FOXA1, FOXC1, or any other marker relevant to prostate cancer. Suitable tissue samples include samples of blood and blood fluids (e.g., serum and plasma), and prostate tissue samples including those that may be obtained by a biopsy or following surgical resection of the prostate, surrounding tissues, and/or distant tissues in which metastasis are known or are suspected.

### BRIEF DESCRIPTION OF THE DRAWINGS

Further aspects of the present disclosure will be more readily appreciated upon review of the detailed description of its various embodiments, described below, when taken in conjunction with the accompanying drawings.

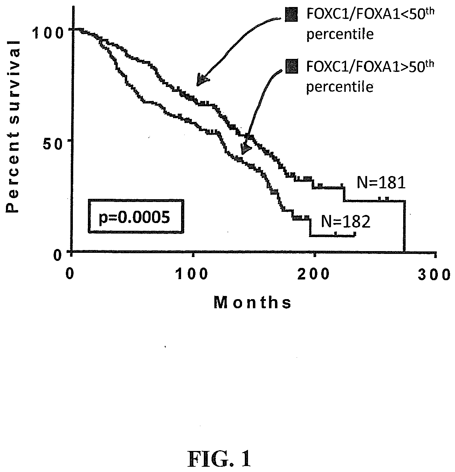

**FIG.** 1 is a line graph showing the survival of patients diagnosed as having prostate cancer, stratified based on whether their FOXC1/FOXA1 ratio is or greater than the FOXC1/FOXA1 ratio that demarcates 50^{th} percentile within the study population, as described in Example 1.

### DETAILED DESCRIPTION

*Prostate Cancer*: Prostate cancer is a major public health challenge, with about 219,000 new cases diagnosed and about 27,000 deaths annually in the United States. Approximately 40-70% of prostate cancers harbor an acquired chromosomal translocation that results in the fusion of the promoter region of the transmembrane protease serine 2 (TMPRSS2) gene to the coding region of members of the erythroblast transformation specific (ETS) family of transcription factors. The most common ETS family member observed with a TMPRSS2 gene translocation is the v-ets erythroblastosis virus E26 oncogene homolog (avian) (ERG) which generally imparts a more aggressive phenotype than prostate cancers of other etiologies.

*FOX Genes*: FOX genes encode a subgroup of helix-tum-helix class of proteins. The arrangement of loops connecting the β strands that flank one of the three α helices, gives rise to a butterfly-like appearance, hence the name 'winged-helix' transcription factors. It is a relatively invariant structure, with most amino acids being conserved between family members. All FOX genes can bind DNA and the functional effect of this can be either the activation or the inhibition of gene transcription. In contrast to the DNA-binding domains, there is almost no sequence homology between the transactivation or repression domains of members of the FOX family, and little is known about their interactions with the transcriptional machinery. The FOX family has been implicated in various cellular processes and they are important in embryonic development and disease. Little is known about the role of the FOX family in the developing and adult prostate. Immunohistochemical localization of FOXA1 reveals epithelial nuclear staining of both members in the developing mouse prostate, but only FOXA1 in the adult mouse prostate. FOXA1 is essential for full prostate ductal morphogenesis as was shown using FOXA1-deficient mice.

The role of various FOX genes in prostate cancer progression might be explained by their interaction with the androgen receptor (AR) pathway. The AR is a nuclear receptor that is activated upon testosterone or dihydrotestosterone binding and generally signals growth of prostate cancer cells [11]. Besides the above mentioned FOXA1 and FOXA2, also FOXG1, FOXH1, FOXO1 and FOXO3 affect the AR cascade. The general theme is that these FOX proteins (all except FOXO3) repress AR activity by directly binding the AR protein. Takayama et al. [12] supported this idea and showed that FOXP1 is an androgen-responsive transcription factor that negatively regulates AR signalling in prostate cancer cells.

The present invention is based on the discovery that the ratio of FOXC1:FOXA1 is a prognostic indicator in patients diagnosed with prostate cancer. In particular, a high FOXC1:FOXA1 ratio indicates a poor prognosis for at least one prognostic outcome. The FOXC1:FOXA1 ratio also may be used to diagnose a prostate cancer and/or differentiate a prostate cancer from benign prostatic hyperplasia (BPH).

### EXAMPLE 1: Identification of the FOXC1:FOXA1 Ratio as a Prognostic Indicator

### Patient Population

In order to obtain a sufficient number of prostate cancer samples, biopsy samples of from two cohorts were combined in this study (see, Setlur et al., J. Natl. Cancer Inst. 100: 815-825, 2008).

*Swedish Cohort:* The population-based Swedish Watchful Waiting Cohort consists of 1256 men with localized prostate cancer. These men had symptoms of benign prostatic hyperplasia (lower urinary tract symptoms) and were subsequently diagnosed with prostate cancer. All men in this study were determined at the time of diagnosis to have clinical stage T1-T2, Mx, N0, according to the 2002 American Joint Commission Committee Tumor-Node-Metastasis staging system (Andren et al., Sweden J. Urol. 175: 1337-1340, 2006; Varenhorst et al., Scand. J. Urol. Nephrol. 39: 117-123, 2005). The prospective follow-up time was up to 30 years. The regional cohort includes men who were diagnosed at University Hospital in Örebro (1977-1991) and at four centers in the southeast region of Sweden: Kalmar, Norrköping, Linköping, and Jonköping (1987-1999). A subset of men from these cohorts (n = 388) were included in the present study. Inclusion criteria required the availability of greater than 90% tumor cells compared with surrounding stroma or benign tissue in the diagnostic Trans Urethral Radical Prostatectomy (TURP) biopsy sample. Samples included were derived from an equal proportion of men who died of prostate cancer or developed metastasis and men who lived a minimum of 10 years without clinical recurrence of their disease. Of these 388 patients, only the 354 with reliable TMPRSS2-ERG fusion results were included in the analyses.

*Physician Health Study (PHS) Prostatectomy Confirmation Cohort:* This cohort included 116 US men who were diagnosed with prostate cancer between 1983 and 2003, and were treated by radical prostatectomy as primary therapy. The men were participants in an ongoing randomized trial in the primary prevention of cancer and cardiovascular disease. Only the 101 patients with reliable TMPRSS2-ERG fusion results were included in the analysis.

### Sample Processing and cDNA-mediated Annealing, Selection, Ligation, and Extension

Foci highly enriched for prostate cancer (>90%) were identified by microscopic examination of the tissue sections by the study pathologists. Three 0.6 µm biopsy cores per patient were taken from these enriched areas and were placed in one well of a 96-well plate for high-throughput RNA extraction. The CyBi-Well liquid handling system (CyBio AG, Jenna, Germany) was used for high-throughput extraction. Cores were first deparaffinized by incubation with 800 µL Citrisolv (Fisher Scientific, USA) at 60°C for 20 minutes and then with 1.2 mL Citrisolv:absolute alcohol (2:1) at room temperature for 10 minutes. Cores were then washed with absolute alcohol, dried at 55°C, and incubated overnight at 45°C in 300 µL lysis buffer (10 mM NaCl, 500 mM Tris pH 7.6, 20 mM EDTA, 1% SDS) containing 1 mg/mL proteinase K (Ambion, Austin, TX). RNA was extracted from the lysate using the TRIzol LS reagent (Invitrogen, Carlsbad, CA). TRIzol LS reagent (900 µL) was added to the cell lysate, followed by 240 µL of chloroform (Sigma-Aldrich, St. Louis, MO). The samples were mixed thoroughly and centrifuged at 4°C, 5600g for 40 minutes (the same centrifugation settings were used for the rest of the protocol). After centrifugation, the aqueous phase was transferred to a new plate, and the RNA was precipitated by incubation with 620 µL of isopropanol (Sigma-Aldrich) at room temperature for 10 minutes. Glycogen (20 µg; Invitrogen) was added as a carrier. The samples were centrifuged as above, and the pellet was washed with 80% ethanol (Sigma-Aldrich), air dried, and dissolved in RNase-free water. The RNA was quantified using a NanoDrop spectrophotometer (NanoDrop technologies, Wilmington, DE).

SYBR green (QIAGEN Inc., Valencia, CA) quantitative polymerase chain reaction (qPCR) assay for a housekeeping gene, ribosomal protein L13a (RPL13A), was used to estimate RNA quality (RNA with crossover threshold, Ct, of less than 30 cycles was considered to be good quality). Primer sequences for RPL13A were as follows: RPL13A-FWD, GTACGCTGTGAAGGCATCAA (SEQ ID NO: 1), and RPL13A-REV, GTTGGTGTTCATCCGCTT (SEQ ID NO: 2) (GenBank accession NM_012423.2). DASL expression assay (Illumina Inc., San Diego, CA) was performed using 50 ng of cDNA according to manufacturer's instructions.

### Determination of TMPRSS2-ERG Fusion Status in Biopsy Samples

TMPRSS2-ERG fusion status was determined by ERG break-apart fluorescence in situ hybridization (FISH) assay and qPCR for cases not assessable by FISH. An aliquot of the RNA used for DASL was used for qPCR. cDNA was synthesized as above using the Illumina kit (Illumina Inc., San Diego, CA). The TMPRSS2-ERG fusion product was detected using SYBR green assay (QIAGEN) with primers directed to the fusion sequence provided at GenBank accession code NM_DQ204772.1 (fusion of TMPRSS2 exon 1 with ERG exon 4). RPL13A was used as a positive control and a calibrator for quantification. Relative quantification was carried out using the comparative ΔΔCt method.

FISH was performed on the 472 prostate cancers for which tissue was available. For samples with inconclusive FISH results, we used qPCR to determine the TMPRSS2-ERG fusion status (455 cancers were successfully annotated, 354 from the Swedish cohort and 101 from the PHS cohort). These experiments indicated that 62 (17.5%) of the prostate tumors of patients in the Swedish Watchful Waiting cohort (diagnosed following transurethral prostate resections for benign prostatic hyperplasia) were positive for the TMPRSS2-ERG fusion. Within the PHS cohort, the majority of cancers (n=83 [82%]) were diagnosed through prostate-specific antigen (PSA) screening, and (n=41, [41%]) of the cancers were positive for TMPRSS2-ERG fusion.

### Determination of Estrogen Receptor Expression in Prostate Cancer Samples

Estrogen receptor-alpha (ERα) and -beta (ERβ) may be determined in prostate cancer tissue samples using quantitative polymerase chain reaction (qPCR), Western blotting, and/or any other suitable quantitative, semi-quantitative, or qualitative technique.

*qPCR*: Total RNA is extracted from the prostate cancer samples and reverse transcribed (RT), with about 50 µg of the resultant cDNAs being subjected to PCR analysis. RT-PCR may be carried out using primers for ERα and/or EPβ (e.g., using primers specific for the target sequences provided at GenBank accession codes NM_000125.2 and NM_001437.2, respectively). cDNA may be synthesized using the Omniscript RT kit (QIAGEN Inc.), and any suitable housekeeping gene may be used for normalization.

*Western Blotting*: Expression of ERα and EPβ may be assessed in prostate cancer tissue samples. Protein extracts may be prepared in RIPA buffer (50 mM Tris pH 7.5, 150 mM NaCl, 2 mM sodium orthovanadate, 0.1% Nonidet P-40, 0.1% Tween 20) with 1x Complete Protease Inhibitor Cocktail (Roche, Indianapolis, IN). Protein concentration may be determined using the Bio-Rad DC protein assay (Bio-Rad Laboratories, Hercules, CA). In one example, equal amounts (e.g., 20 µg) of total protein are loaded on NuPAGE 4-12% Tris-Bis gels (Invitrogen) and transferred to Immobilon-P polyvinylidene fluoride membranes (Millipore, Billerica, MA). Blots then may be incubated with primary antibodies (e.g., mouse monoclonal anti-ERa [1:100, NeoMarkers, Labvision Corporation, Fremont, CA] or mouse monoclonal anti-ERβ [1:200, clone 14C8, GeneTex Inc., San Antonio, TX]), washed three times with PBS containing 0.1% Triton X-100, and detected using any suitable detection methodology (e.g., incubated with peroxidase-conjugated anti-mouse secondary antibody (1:8000, Amersham Biosciences, Piscataway, NJ), for 1 hour). β-actin, or any other suitable housekeeping protein may be used as a control for protein loading and transfer. Antibody-protein complexes may be detected using any suitable means including, for example, the ECL Western Blotting Analysis System (Amersham Biosciences, Piscataway, NJ).

### cDNA-mediated Annealing, Selection, Ligation, and Extension Array Design

A set of four cDNA-mediated annealing, selection, ligation, and extension (DASL) Assay Panels (DAPs) for the discovery of molecular signatures relevant to prostate cancer was developed. Informative genes, i.e., genes showing differential expression across samples in previously generated microarray data sets (the datasets are at http://www.broad.mit.edu/cancer/pub/HCC) were prioritized which included 24 studies, 2149 samples, and 15 tissue types. The top-ranked transcriptionally informative genes that showed the largest variation in expression across the different datasets comprised genes in most of the known biological pathways. To ensure that prostate cancer-related genes were included in the DAP, a meta-analysis of previous microarray datasets from the Oncomine Database was performed and included from that a list of genes that were transcriptionally regulated in prostate cancer. The final array consisted of 6144 genes (6K DAP).

Thus, a high-throughput method to profile the expression of 6144 genes in archival tissue specimens was developed. High-quality expression data were obtained from 472 of 504 (93.65%) of the prostate cancer samples (363 from the Swedish cohort and 109 from the PHS cohort). The data have been deposited in NCBI's Gene Expression Omnibus (GEO, http://www.ncbi.nlm.nih.gov/geo/) and are accessible through GEO series accession number GSE8402.

The expression level of FOXA1 and FOXC1 were identified and determined in 363 samples and the median ratio determined for the population (i.e., cutoff for the 50^{th} percentile). A survival curve for the population having a FOXC1:FOXA1 ratio below the median was generated and plotted against the survival curve for the population having a FOXC1:FOXA1 ratio above the median. As shown in Figure 1, prostate cancer patients in the lower FOXC1:FOXA1 ratio group had a significantly better prognosis, in terms of overall survival duration, compared to patients in the higher FOXC1:FOXA1 ratio group (p=0.0005). This demonstrates that the FOXC1:FOXA1 ratio is a useful prognostic indicator for patients diagnosed as having prostate cancer.

## Claims

1. A method for determining the prognosis of a prostate cancer in a subject diagnosed as having prostate cancer, comprising:
(i) determining in a tissue sample from said subject, wherein said tissue sample comprises prostate cancer cells, the level of FOXA1 protein or nucleic acid, wherein the FOXA1 nucleic acid is optionally FOXA1 mRNA,
(ii) determining the level of FOXC1 protein or nucleic acid in said tissue sample, wherein the FOXC1 nucleic acid is optionally FOXC1 mRNA,
(iii) calculating the ratio of said FOXC1 protein or nucleic acid to said FOXA1 protein or nucleic acid;
(iv) comparing said ratio to a comparable FOXC1:FOXA1 ratio in a reference population of samples comprising prostate cancer cells in which a prognostic outcome is associated with each of the reference population samples; and
(v) identifying said subject as having a poor prognosis relative to the prognostic outcome when the FOXC1:FOXA1 ratio of the subject is equal to or greater than a FOXC1:FOXA1 cutoff ratio, and identifying said subject as having a good prognosis relative to the prognostic outcome when the FOXC1:FOXA1 ratio of the subject is less than the FOXC1:FOXA1 cutoff ratio, wherein the FOXC1:FOXA1 cutoff ratio corresponds to at least the 50^{th} percentile of FOXC1:FOXA1 ratios of the reference population.

2. The method of claim 1, wherein the prostate cancer comprises a cancer selected from the group consisting of an adenocarcinoma, a small cell carcinoma, and a prostatic sarcoma.

3. The method claim 1 or 2, wherein the FOXC1:FOXA1 cutoff ratio corresponds to at least the 90^{th} percentile of FOXC1:FOXA1 ratios of the reference population.

4. The method of any one of claims 1-3, wherein the prognostic outcome is selected from the group consisting of survival duration, event-free survival, and relapse-free survival.

5. The method of any one of claims 1-4, wherein said method further comprises:
(vi) developing a treatment plan based on the prognostic identification of step (v).

6. The method of claim 5, wherein the treatment plan comprises (a) discontinuing at least one anti-cancer therapy for a subject having a good prognosis, (b) maintaining at least one anti-cancer therapy for a subject having a good prognosis, (c) initiating at least one anti-cancer therapy for a subject having a good prognosis, (d) discontinuing at least one anti-cancer therapy for a subject having a poor prognosis, or (e) initiating at least one anti-cancer therapy for a subject having a poor prognosis.

7. The method of claim 5, wherein the treatment plan comprises increasing the frequency, duration, or dose of at least one anti-cancer therapy for a subject having a poor prognosis.

8. A method for diagnosing a prostate cancer in a subject, comprising:
(i) determining in tissue sample from said subject, wherein said tissue sample is suspected of comprising prostate cancer cells the level of FOXA1 protein or nucleic acid, wherein the FOXA1 nucleic acid is optionally FOXA1 mRNA,
(ii) determining the level of FOXC1 protein or nucleic acid in said tissue sample, wherein the FOXC1 nucleic acid is optionally FOXC1 mRNA,
(iii) calculating the ratio of said FOXC1 protein or nucleic acid to said FOXA1 protein or nucleic acid;
(iv) comparing said ratio to a comparable FOXC1:FOXA1 ratio in a reference population of samples known to comprise prostate cancer cells; and
(v) identifying said subject as having a prostate cancer when the FOXC1 :FOXA1 ratio of the subject is equal to or greater than a FOXC1 :FOXA1 cutoff ratio, and identifying said subject as not having a prostate cancer when the FOXC1 :FOXA1 ratio of the subject is less than the FOXC1:FOXA1 cutoff ratio, wherein the FOXC1:FOXA1 cutoff ratio corresponds to at least the 20^{th} percentile of FOXC1:FOXA1 ratios of the reference population.

9. The method of claim 8, wherein the prostate cancer comprises a cancer selected from the group consisting of an adenocarcinoma, a small cell carcinoma, and a prostatic sarcoma.

10. The method of claim 9, wherein the FOXC1:FOXA1 cutoff ratio corresponds to at least the 75^{th} percentile of FOXC1:FOXA1 ratios of the reference population.

11. The method claim 9 or 10, wherein said method further comprises:
(vi) developing a treatment plan based on the diagnostic identification of step (v).

12. The method of claim 11, wherein the treatment plan comprises (a) initiating radiation therapy, (b) initiating chemotherapy, or (c) a partial surgical resection of the prostate cancer.

## Patentansprüche

1. Verfahren zum Stellen der Prognose eines Prostatakrebses bei einem Patienten, bei dem Prostatakrebs diagnostiziert wurde, umfassend:
(i) Bestimmen in einer Gewebeprobe von dem Patienten, wobei die Gewebeprobe Prostatakrebszellen umfasst, des Gehalts an FOXA1-Protein oder-Nukleinsäure, wobei die FOXA1-Nukleinsäure wahlweise FOXA1 mRNA ist,
(ii) Bestimmen des Gehalts an FOXC1-Protein oder -Nukleinsäure in der Gewebeprobe, wobei die FOXC1-Nukleinsäure wahlweise FOXC1 mRNA ist,
(iii) Berechnen des Verhältnisses des FOXC1-Proteins oder der -Nukleinsäure zu dem FOXA1-Protein oder der -Nukleinsäure;
(iv) Vergleichen des Verhältnisses mit einem vergleichbaren FOXC1:FOXA1-Verhältnis in einer Referenzpopulation von Proben, die Prostatakrebszellen aufweisen, wobei ein Prognoseergebnis mit jeder der Referenzpopulationsproben verknüpft ist, und
(v) Feststellen, dass der Patient in Bezug auf das Prognoseergebnis eine schlechte Prognose hat, wenn das FOXC1:FOXA1-Verhältnis des Patienten gleich oder größer ist als ein FOXC1:FOXA1 Cutoff-Verhältnis, und Feststellen, dass der Patient in Bezug auf das Prognoseergebnis eine gute Prognose hat, wenn das FOXC1:FOXA1-Verhältnis des Patienten niedriger ist als das FOXC1:FOXA1-Cutoff-Verhältnis, wobei das FOXC1:FOXA1-Cutoff-Verhältnis mindestens dem 50. Perzentil der FOXC1:FOXA1-Verhältnisse der Referenzpopulation entspricht.

2. Verfahren nach Anspruch 1, wobei der Prostatakrebs einen Krebs umfasst, ausgewählt aus der Gruppe, bestehend aus einem Adenokarzinom, einem kleinzelligen Karzinom und einem Prostatasarkom.

3. Verfahren nach Anspruch 1 oder 2, wobei das FOXC1:FOXA1-Cutoff-Verhältnis mindestens dem 90. Perzentil der FOXC1:FOXA1-Verhältnisse der Referenzpopulation entspricht.

4. Verfahren nach einem der Ansprüche 1-3, wobei das Prognoseergebnis ausgewählt ist aus der Gruppe, bestehend aus Überlebensdauer, ereignisfreies Überleben und rezidiv-freies Überleben.

5. Verfahren nach einem der Ansprüche 1-4, wobei das Verfahren weiterhin Folgendes umfasst:
(vi) Erstellen eines Behandlungsplans, basierend auf der Prognosefeststellung von Schritt (v).

6. Verfahren nach Anspruch 5, wobei der Behandlungsplan Folgendes umfasst: (a) Beenden mindestens einer Anti-Krebstherapie bei einem Patienten, der eine gute Prognose hat, (b) Aufrechterhalten mindestens einer Anti-Krebstherapie bei einem Patienten, der eine gute Prognose hat, (c) Beginnen mindestens einer Anti-Krebstherapie bei einem Patienten, der eine gute Prognose hat, (d) Beenden mindestens einer Anti-Krebstherapie bei einem Patienten, der eine schlechte Prognose hat, oder (e) Beginnen mindestens einer Anti-Krebstherapie bei einem Patienten, der eine schlechte Prognose hat.

7. Verfahren nach Anspruch 5, wobei der Behandlungsplan das Erhöhen der Häufigkeit, Dauer oder Dosis von mindestens einer Anti-Krebstherapie bei einem Patienten umfasst, der eine schlechte Prognose hat.

8. Verfahren zur Diagnose eines Prostatakrebses bei einem Patienten, umfassend:
(i) Bestimmen in einer Gewebeprobe von dem Patienten, wobei von der Gewebeprobe vermutet wird, dass sie Prostatakrebszellen umfasst, des Gehalts an FOXA1-Protein oder -Nukleinsäure, wobei die FOXA1 -Nukleinsäure wahlweise FOXA1 mRNA ist,
(ii) Bestimmen des Gehalts an FOXC1-Protein oder -Nukleinsäure in der Gewebeprobe, wobei die FOXC1-Nukleinsäure wahlweise FOXC1 mRNA ist,
(iii) Berechnen des Verhältnisses des FOXC1-Proteins oder der -Nukleinsäure zu dem FOXA1-Protein oder der -Nukleinsäure;
(iv) Vergleichen des Verhältnisses mit einem vergleichbaren FOXC1 :FOXA1-Verhältnis in einer Referenzpopulation von Proben, die bekanntlich Prostatakrebszellen aufweisen, und
(v) Feststellen, dass der Patient Prostatakrebs hat, wenn das FOXC1 :FOXA1-Verhältnis des Patienten gleich oder größer ist als ein FOXC1 :FOXA1-Cutoff-Verhältnis, und Feststellen, dass der Patient keinen Prostatakrebs hat, wenn das FOXC1 :FOXA1-Verhältnis des Patienten niedriger ist als das FOXC1 :FOXA1-Cutoff-Verhältnis, wobei das FOXC1 :FOXA1-Cutoff-Verhältnis mindestens dem 20. Perzentil der FOXC1 :FOXA1 -Verhältnisse der Referenzpopulation entspricht.

9. Verfahren nach Anspruch 8, wobei der Prostatakrebs einen Krebs umfasst, ausgewählt aus der Gruppe, bestehend aus einem Adenokarzinom, einem kleinzelligen Karzinom und einem Prostatasarkom.

10. Verfahren nach Anspruch 9, wobei das FOXC1:FOXA1-Cutoff-Verhältnis mindestens dem 75. Perzentil der FOXC1:FOXA1 -Verhältnisse der Referenzpopulation entspricht.

11. Verfahren nach Anspruch 9 oder 10, wobei das Verfahren weiterhin Folgendes umfasst:
(vi) Erstellen eines Behandlungsplans, basierend auf der Diagnosefeststellung von Schritt (v).

12. Verfahren nach Anspruch 11, wobei der Behandlungsplan Folgendes umfasst: (a) Beginnen einer Strahlentherapie, (b) Beginnen einer Chemotherapie oder (c) teilweise operative Entfernung des Prostatakrebses.

## Revendications

1. Procédé de détermination d'un pronostic pour un cancer de la prostate dans un sujet diagnostiqué comme ayant le cancer de la prostate, comprenant:
(i) déterminer dans un échantillon de tissu dudit sujet, ledit échantillon de tissu comprenant des cellules du cancer de la prostate, le niveau de la protéine ou de l'acide nucléique FOXA1, l'acide nucléique FOXA1 étant facultativement FOXA1 ARNm,
(ii) déterminer le niveau de la protéine ou de l'acide nucléique FOXC1 dans ledit échantillon de tissu, l'acide nucléique FOXC1 étant facultativement FOXC1 ARNm,
(iii) calculer le rapport de ladite protéine ou dudit acide nucléique FOXC1 à ladite protéine ou audit acide nucléique FOXA1;
(iv) comparer ledit rapport à un rapport comparable FOXC1:FOXA1 dans une population de référence des échantillons comprenant des cellules du cancer de la prostate dans laquelle un résultat de pronostic est associé avec chacun des échantillons de la population de référence; et
(v) identifier ledit sujet comme ayant un mauvais pronostic relatif au résultat de pronostic lorsque le rapport FOXC1:FOXA1 du sujet est égal ou supérieur au rapport de coupure FOXC1:FOXA1, et identifier ledit sujet comme ayant un bon pronostic relatif au résultat de pronostic lorsque le rapport FOXC1:FOXA1 du sujet est inférieur au rapport de coupure FOXC1:FOXA1, le rapport de coupure FOXC1:FOXA1 correspondant au moins au 50^{e} percentile des rapports FOXC1:FOXA1 de la population de référence.

2. Procédé selon la revendication 1, le cancer de la prostate comprenant un cancer choisi parmi le groupe constitué d'un adénocarcinome, d'un carcinome à petites cellules et d'un sarcome de la prostate.

3. Procédé selon la revendication 1 ou 2, le rapport de coupure FOXC1:FOXA1 correspondant au moins au 90^{e} percentile des rapports FOXC1:FOXA1 de la population de référence.

4. Procédé selon l'une quelconque des revendications 1-3, le résultat de pronostic étant choisi parmi le groupe constitué de la durée de survie, de la survie sans évènement et de la survie sans rechute.

5. Procédé selon l'une quelconque des revendications 1-4, ledit procédé comprenant en outre:
(vi) développer un plan de traitement basé sur l'identification pronostique de l'étape (v).

6. Procédé selon la revendication 5, le plan de traitement comprenant (a) cesser au moins une thérapie anticancéreuse pour un sujet ayant un bon pronostic, (b) maintenir au moins une thérapie anticancéreuse pour un sujet ayant un bon pronostic, (c) initier au moins une thérapie anticancéreuse pour un sujet ayant un bon pronostic, (d) cesser au moins une thérapie anticancéreuse pour un sujet ayant un mauvais pronostic ou (e) initier au moins une thérapie anticancéreuse pour un sujet ayant un mauvais pronostic.

7. Procédé selon la revendication 5, le plan de traitement comprenant l'augmentation de la fréquence, de la durée ou de la dose au moins d'une thérapie anticancéreuse pour un sujet ayant un mauvais pronostic.

8. Procédé pour diagnostiquer un cancer de la prostate dans un sujet, comprenant:
(i) déterminer dans un échantillon de tissu dudit sujet, ledit échantillon de tissu étant suspecté de comprendre des cellules du cancer de la prostate, le niveau de la protéine ou de l'acide nucléique FOXA1, l'acide nucléique FOXA1 étant facultativement FOXA1 ARNm,
(ii) déterminer le niveau de la protéine ou de l'acide nucléique FOXC1 dans ledit échantillon de tissu, l'acide nucléique FOXC1 étant facultativement FOXC1 ARNm,
(iii) calculer le rapport de ladite protéine ou dudit acide nucléique FOXC1 à ladite protéine ou audit acide nucléique FOXA1;
(iv) comparer ledit rapport à un rapport comparable FOXC1:FOXA1 dans une population de référence des échantillons connus de comprendre des cellules du cancer de la prostate, et
(v) identifier ledit sujet comme ayant un cancer de la prostate lorsque le rapport FOXC1:FOXA1 du sujet est égal ou supérieur à un rapport de coupure FOXC1:FOXA1, et identifier ledit sujet comme n'ayant pas un cancer de la prostate lorsque le rapport FOXC1:FOXA1 du sujet est inférieur au rapport de coupure FOXC1:FOXA1, le rapport de coupure FOXC1:FOXA1 correspondant au moins au 20^{e} percentile des rapports FOXC1:FOXA1 de la population de référence.

9. Procédé selon la revendication 8, le cancer de la prostate comprenant un cancer choisi parmi le groupe constitué d'un adénocarcinome, d'un carcinome à petites cellules et d'un sarcome de la prostate.

10. Procédé selon la revendication 9, le rapport de coupure FOXC1:FOXA1 correspondant au moins au 75^{e} percentile des rapports FOXC1:FOXA1 de la population de référence.

11. Procédé selon la revendication 9 ou 10, ledit procédé comprenant en outre:
(vi) développer un plan de traitement basé sur l'identification diagnostique de l'étape (v).

12. Procédé selon la revendication 11, le plan de traitement comprenant (a) initier une radiothérapie, (b) initier une chimiothérapie ou (c) une résection chirurgicale partielle du cancer de la prostate.
